# FASCICULE DE BREVET EUROPEEN

(11) **EP 4 231 916 B1**
(45) Date de publication et mention de la délivrance du brevet: **09.07.2025**
(21) Numéro de dépôt: 21801477.7
(22) Date de dépôt: 26.10.2021
(51) Int. Cl.: A61B 6/02, A61B 6/00, A61B 6/03

(54) **DISPOSITIF DE RADIOLOGIE A SOURCES ET DETECTEUR DISPOSES EN HELICE**
RADIOLOGIE-VORRICHTUNG AUFWEISEND EINE QUELLE UND DETEKTOR IN SPIRALFÖRMIGER ANORDNUNG
RADIOLOGY DEVICE WITH SOURCES AND DETECTOR DISPOSED IN HELICAL CONFIGURATION

(30) Priorité: 26.10.2020 FR 2010947
(43) Date de publication de la demande: 30.08.2023
(73) Titulaire: THALES, 92400 Courbevoie (FR)
(72) Inventeur: BERNARD, Guillaume, 38430 Moirans (FR); ROYER, Guillaume, 38430 Moirans (FR)
(74) Mandataire: Atout PI Laplace
(86) Numéro de dépôt international: PCT/EP2021/079726
(87) Numéro de publication internationale: WO 2022/090257

(56) Documents cités:
- US-A1- 2003 161 434
- US-A1- 2005 111 610

## Description

L'invention concerne un dispositif de radiologie. L'invention peut être mise en œuvre dans le domaine médical, dans l'industrie pour réaliser des contrôles non destructifs et dans la sécurité pour détecter des objets ou des matériaux dangereux. L'invention trouve une utilité particulière en tomodensitométrie.

De façon connue, la tomodensitométrie, également appelée scanographie met en œuvre un système équipé d'un tube à rayons X émettant un faisceau collimaté en forme d'éventail connu en anglais sous le nom de « fan beam ». Le tube à rayons X est associé à un détecteur disposé en regard du faisceau. Le tube et le détecteur tournent autour d'une table recevant le patient. A chaque tour, la table avance en suivant l'axe de rotation du tube et du détecteur d'un petit décalage correspondant à l'épaisseur d'une coupe patient. Un traitement informatique permet de reconstruire des coupes 2D ou volume 3D des structures anatomiques du patient. Ce système est connu sous le nom de « CT-scanner ». « CT » étant l'acronyme anglais de « Computer Tomography ».

Les équipements mécaniques permettant de faire tourner le tube et le détecteur sont volumineux et lourds.

L'invention vise à proposer un dispositif de radiologie pouvant réaliser des examens de tomodensitométrie en évitant de mettre en œuvre un tube et un détecteur tournant ensemble. Un système de l'art antérieur comprenant une source et un détecteur tous deux fixes est décrit dans le document de brevet US 2003/161434 A1.

A cet effet, l'invention a pour objet un dispositif de radiologie comprenant un support mobile en translation selon un axe de translation par rapport à un bâti du dispositif, le support étant destiné à porter un objet à imager, un générateur de rayons ionisants et un détecteur configuré pour détecter les rayons émis par le générateur, le générateur et le détecteur étant en regard l'un par rapport à l'autre. Le générateur comprend plusieurs sources dont on considère qu'elles émettent chacune à partir d'un point de focalisation, les points de focalisation des différentes sources étant répartis le long d'un axe des sources, le détecteur s'étendant selon un axe du détecteur, l'axe des sources et l'axe du détecteur s'étendant en forme d'hélice imbriquées l'une dans l'autre autour de l'axe de translation. Chacune des sources émet un faisceau de rayons ionisants sensiblement plat et en forme d'éventail. Le dispositif comprend en outre un collimateur possédant, en regard de chaque source, une fente configurée pour obtenir le faisceau en forme d'éventail réalisée dans un plan du collimateur parallèle à l'axe de translation. Le collimateur s'étend principalement selon un axe du collimateur parallèle à l'axe des sources. Les fentes ne sont pas sécantes. Les fentes s'étendent principalement chacune selon une portion de courbe définie dans le plan du collimateur et des projections perpendiculairement à l'axe du collimateur, de portions de courbes, correspondant à des fentes consécutives, sur l'axe du collimateur possèdent une partie commune.

Avantageusement, le générateur et le détecteur sont fixes par rapport au bâti.

Les formes en hélice sont avantageusement positionnées entre elles de façon à ce que pour chacune des sources un faisceau de rayons ionisants issu de la source considérée et atteignant une portion du détecteur soit sensiblement situé dans un plan perpendiculaire à l'axe de translation.

Les portions de courbes suivent avantageusement sensiblement une forme sinusoïdale.

Avantageusement, les portions de courbes comprennent au moins une portion de droite approximant la forme sinusoïdale.

Avantageusement, les portions de courbe des différentes fentes s'étendent parallèlement entre elles.

Le détecteur est avantageusement disposé en partie entre le générateur et l'axe de translation dans un plan perpendiculaire à l'axe de translation tout en laissant un passage libre pour les rayons émis par le générateur en direction du détecteur.

La forme en hélice de l'axe des sources et la forme en hélice de l'axe du détecteur s'appuient avantageusement chacune sur une courbe régulière centrée sur l'axe de translation.

Les courbes régulières sont avantageusement formées chacune sur une surface cylindrique à section circulaire ou à section polygonale régulière dont l'axe est l'axe de translation.

Chaque source comprend avantageusement une cathode froide émettant un faisceau d'électrons par effet de champ.

L'invention sera mieux comprise et d'autres avantages apparaîtront à la lecture de la description détaillée d'un mode de réalisation donné à titre d'exemple, description illustrée par le dessin joint dans lequel :
la figure 1 représente schématiquement un dispositif de radiologie selon l'invention ;
la figure 2 représente en vue de profil un premier mode de réalisation d'une association d'un détecteur et de plusieurs sources du dispositif de radiologie représenté sur la figure 1 ;
la figure 3 représente en perspective un second mode de réalisation d'une association d'un détecteur et de plusieurs sources du dispositif de radiologie représenté sur la figure 1 ;
la figure 4 représente un exemple de générateur de rayons ionisants pouvant être mis en œuvre dans un dispositif de radiologie conforme à l'invention ;
la figure 5 représente l'association d'un détecteur, de plusieurs sources et d'un premier exemple de collimateur ;
Les figures 6a, 6b et 6c représentent plusieurs exemples de formes de fentes du collimateur de la figure 5 ;
la figure 7 représente l'association d'un détecteur, de plusieurs sources et d'un deuxième exemple de collimateur ;
la figure 8 représente une variante d'une association d'un détecteur et d'un générateur présentant une meilleure imbrication ;
la figure 9 représente le détecteur et le générateur de la figure 7 associé au collimateur de la figure 5.

Par souci de clarté, les mêmes éléments porteront les mêmes repères dans les différentes figures.

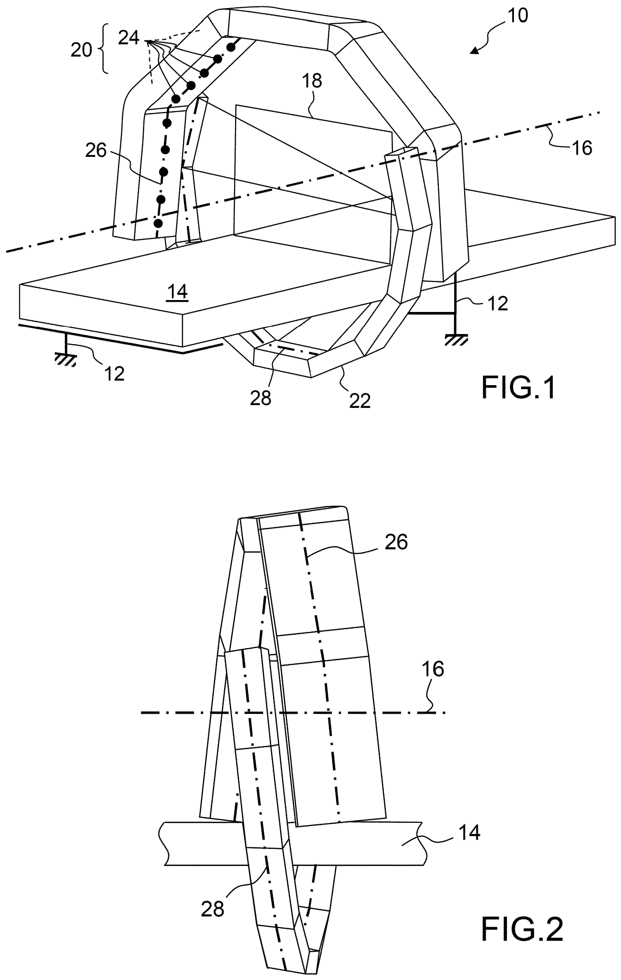

La figure 1 représente un dispositif de radiologie 10 pouvant réaliser des examens de tomodensitométrie. Le dispositif 10 comprend un bâti 12 et un support 14 mobile en translation par rapport au bâti 12. Le support 14 possède ici la forme d'un plateau. Le support 14 est mobile selon un axe de translation 16 par rapport au bâti 12. Un volume utile 18 à section rectangulaire est défini au-dessus du support 14. Le volume utile 18 est destiné à recevoir un objet à imager. Toute autre forme de volume utile est également possible, notamment un volume à section circulaire. La forme du support 14 est alors adaptée en conséquence. Le dispositif 10 comprend un générateur 20 de rayons ionisants et un détecteur 22 associés entre eux. Le générateur 20 et le détecteur 22 sont solidaires du bâti 12. Le détecteur 22 est configuré pour détecter les rayons émis par le générateur 20. Le générateur 20 et le détecteur 22 sont en regard l'un de l'autre afin que le détecteur 22 puisse recevoir les rayons ionisants émis par le générateur 20.

Pour éviter de faire tourner le générateur 20 et le détecteur 22 autour de l'axe 16, le générateur 20 comprend plusieurs sources 24 de rayonnement ionisant statiques réparties autour de l'axe 16. Les différentes sources 24 émettent en direction du détecteur 22. L'émission des sources traversent le volume utile 18 avant d'atteindre le détecteur 22. Pour simplifier, on considère que chacune des sources 24 possède un point de focalisation à partir duquel un faisceau de rayons ionisants s'étend. Sur la figure 1, le point de focalisation de chaque source porte également le repère 24.

Les points de focalisation des différentes sources 24 sont répartis le long d'un axe des sources 26. Par ailleurs, le détecteur 22 s'étend selon un axe du détecteur 28. Le détecteur 22 comprend un ensemble de pixels sensibles au rayonnement émis par les sources éventuellement par l'intermédiaire d'un scintillateur. Les pixels sont répartis en une ou plusieurs rangées s'étendant selon l'axe 28.

La figure 2 représente en vue de profil le générateur 20 et le détecteur 22 représentés sur la figure 1. L'axe de translation 16 est horizontal sur la figure 2. La figure 2 permet d'illustrer plus facilement la forme des axes 26 et 28. Plus précisément, les axes 26 et 28 s'étendent chacun en forme d'hélice autour de l'axe de translation 16. Les formes en hélice de chacun des axes 26 et 28 sont imbriquées l'une dans l'autre. On entend par formes imbriquées deux formes d'hélice déphasées de 180°, l'une pour le générateur 20 et l'autre pour le détecteur 22. Autrement dit, Pour chaque point de l'hélice du générateur correspond un point de l'hélice du détecteur. Ces deux points sont joints par un segment de droite perpendiculaire à l'axe de translation 16. L'imbrication des formes en hélice permet à chacune des sources d'émettre un faisceau sensiblement plat et en forme d'éventail en direction du détecteur 22. La quasi planéité du faisceau émis par chacune des sources 24 facilite la reconstruction en deux ou trois dimensions de l'image radiologique. Pour faciliter la reconstruction de l'image radiologique, les sources 24 sont régulièrement réparties le long de l'axe des sources 26. Les sources 24 du générateur 20 et le détecteur 22 couvrent avantageusement un secteur angulaire compris entre 180° et 270° au tour de l'axe 16.Toujours pour faciliter la reconstruction de l'image radiologique, les formes en hélice sont avantageusement positionnées entre elles de façon à ce que le faisceau issu d'une source et atteignant une portion du détecteur soit sensiblement situé dans un plan perpendiculaire à l'axe de translation 16. Une inclinaison du faisceau par rapport à un plan perpendiculaire à l'axe de translation 16 est possible mais complique la reconstruction de l'image radiologique. Plus le pas de l'hélice est faible, plus le faisceau peut se rapprocher du plan perpendiculaire à l'axe de translation 16.

En pratique, pour mettre en œuvre un faisceau totalement plan entre chacune des sources 24 et le détecteur 22, il faudrait que les sources 24 et le détecteur 22 soient positionnés dans un plan perpendiculaire à l'axe de translation 16. Ceci est impossible si on souhaite couvrir un secteur angulaire supérieur à 180°. Une réalisation en hélice imbriquée avec un pas d'hélice non nul permet de mettre en œuvre un faisceau dont la forme suit une légère sinusoïde par rapport à un plan parfait. Cette sinusoïde est d'autant plus plate que les pas des hélices sont faibles, ce qui est souhaitable. En effet, il est possible d'approximer la sinusoïde par une droite par exemple au moyen d'un développement de Taylor au premier ordre. L'approximation est d'autant meilleure que le pas des hélices est faible. Pour atteindre cet objectif, les composants du générateur 20 et du détecteur 22 seront choisis pour réduire ce pas la plus possible.

Une hélice est une courbe dont la tangente en chaque point fait un angle constant avec une direction donnée, en l'occurrence la direction suivie par l'axe de translation 16. Les hélices sur lesquelles s'appuient les axes 26 et 28 sont formées sur la figure 2 sur des surfaces cylindriques d'axe 16 et à section polygonale proche d'un cercle. Sur la figure 2, les surfaces cylindriques ont une même distance à l'axe 16. Dans le cadre de l'invention les distances à l'axe 16 des deux surfaces cylindriques peuvent être différentes et peuvent varier notamment en fonction des dimensions physiques de chacune des sources 24. De même, les pas des deux hélices est avantageusement le même, ce qui simplifie la reconstruction des images. Pour des raisons de disposition physique du générateur 20 et du détecteur 22, on peut aussi mettre en œuvre l'invention avec un pas d'hélice différent pour l'axe 26 des sources et l'axe 28 du détecteur.

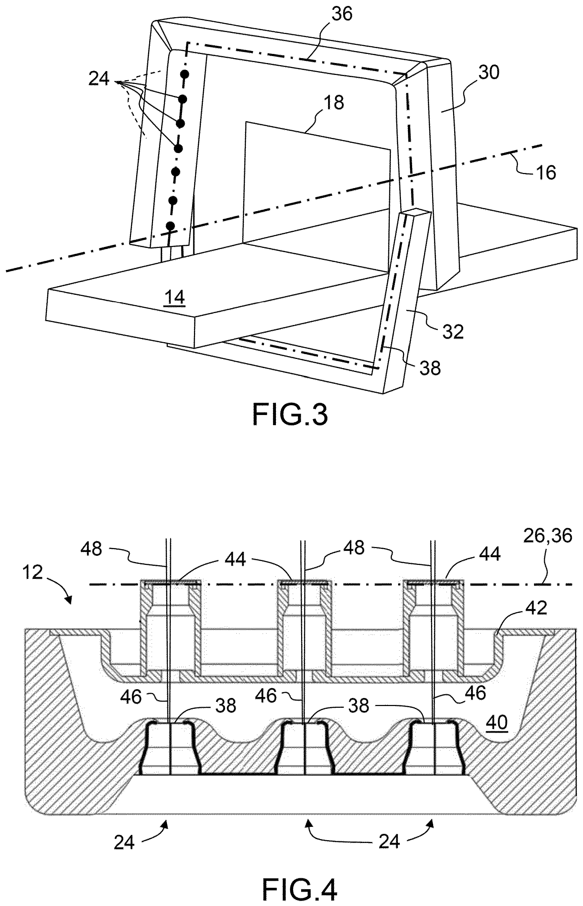

La figure 3 représente un autre mode de réalisation d'un générateur 30 et d'un détecteur 32 également associés entre eux. Comme pour le générateur 20, le générateur 30 comprend plusieurs sources 24 réparties le long d'un axe des sources 36. Par ailleurs, le détecteur 32 s'étend selon un axe du détecteur 38. Les axes 36 et 38 s'étendent également chacun en forme d'hélice autour de l'axe de translation 16. A la différence de la figure 2, dans le mode de réalisation de la figure 3, les hélices sur lesquelles s'appuient les axes 36 et 38 sont formées sur des surfaces cylindriques d'axe 16 et à section sensiblement rectangulaire. Les portions de droite formant la section rectangulaire peuvent faciliter la réalisation du générateur 30 et du détecteur 32. Toute autre forme de surface cylindrique sur lesquelles s'appuient l'axe des sources et l'axe du détecteur est possible dans le cadre de l'invention. Des surfaces cylindriques différentes pour l'axe des sources et pour l'axe du détecteur est également possible. Par exemple, le détecteur peut s'étendre selon un axe s'appuyant sur une surface à section rectangulaire, tandis que le générateur peut s'étendre selon un axe s'appuyant sur une surface à section circulaire. La section peut être par exemple un polygone régulier centré sur l'axe 16. L'intérêt d'un polygone régulier, d'une section circulaire, ou plus généralement une courbe régulière centrée sur l'axe 16 est de conserver une distance moyenne entre les sources et le détecteur sensiblement constante, ce qui facilite la reconstruction de l'image radiologique en deux ou trois dimensions.

En pratique, la mise en œuvre d'hélices s'appuyant sur des surfaces cylindriques à section circulaire reste la solution géométrique optimale pour conserver une distance constante entre les sources et le détecteur. Cependant, il peut être plus aisé de réaliser des portions de droite tel que proposé sur la figure 3. La section rectangulaire forme une approximation grossière de la section circulaire et les sections polygonales régulières à plus de quatre cotés forment de meilleures approximations.

Les sources 24 sont avantageusement compactes comme par exemple décrites dans la demande de brevet publiée sous le n° : WO 2019/011980 A1 et déposée au nom de la demanderesse. Chaque source comprend dans une enceinte à vide, une cathode émettant un faisceau d'électrons, une anode possédant une cible bombardée par le faisceau d'électrons et émettant un faisceau de rayons ionisants. La cathode émet avantageusement le faisceau d'électrons par effet de champ en direction de la cible. Ce type de cathode est également connu sous le nom de cathode froide en opposition aux cathodes chaudes également appelées : cathodes thermoïoniques.

L'intérêt de mettre en œuvre des sources compactes à cathode froide est de permettre la réduction du pas d'hélice de l'axe sources 26 ou 36 et le rapprochement de leur point de focalisation le long de l'axe sources 26 ou 36. En effet, en tomodensitométrie classique où le générateur et le détecteur tournent autour de l'axe de translation, il est possible de réaliser autant d'images que souhaité lors de la rotation afin de disposer de données suffisantes pour la reconstruction d'une image en deux ou trois dimensions. Dans l'invention, en rapprochant les sources 24 entre elles, il est possible de disposer de données suffisantes sans rotation du générateur autour de l'axe 16. Il est cependant possible d'intégrer au dispositif de radiologie un mécanisme permettant la rotation soit du générateur, soit du détecteur soit des deux de façon combinée. Cette rotation est par exemple souhaitable si le générateur ne couvre pas un secteur angulaire d'au moins 180° autour de l'axe 16. La rotation peut ne couvrir qu'une fraction de tout autour de l'axe 16. Il reste cependant avantageux de réaliser un dispositif de radiologie dans lequel le générateur 20 ou 30 et le détecteur 22 ou 32 sont fixes par rapport au bâti 12 du dispositif 10.

La figure 4 représente plus en détail un ensemble de sources 24 possèdent une enceinte à vide 40 commune. Il est notamment possible de réaliser toutes les sources 24 ou tout au moins plusieurs d'entre elles en dans une enceinte à vide 40 unique. L'intérêt d'une enceinte à vide commune à plusieurs sources 24 est de permettre le rapprochement des points focaux des faisceaux émis par chacune des sources 24. La répartition des sources 24 le long de l'axe 26 ou 36 peut être uniforme comme représenté sur la figure 4 où la distance séparant deux sources 24 voisine est constante. Il est également possible d'opter pour une répartition non uniforme. Alternativement, dans le cadre de l'invention il est bien entendu possible de mette en œuvre une enceinte à vide par source 24.

Sur la figure 4, des cathodes froides 38 sont réparties parallèlement à l'axe 26 ou 36. Les sources 24 peuvent comprendre une anode 42 commune aux différentes sources 24. L'anode 42 porte autant de cibles 44 que de cathodes 38. Chaque cathode 38 émet un faisceau d'électrons 46 en direction de la cible 44 qui lui est associée. L'interaction entre un faisceau d'électrons 46 et une cible 44 permet de générer un faisceau de rayons ionisant 48. Les différentes sources 24 peuvent être pilotées indépendamment les unes des autres au moyen du pilotage de leur cathode respective 38. Dans le mode de réalisation décrit à l'aide de la figure 3 où l'axe 36 est formé de plusieurs segments de droite, les sources 24 disposées sur un même segment peuvent avoir une enceinte à vide 40 commune.

Il est bien entendu que l'invention peut également être mise en œuvre avec des sources à cathodes thermoïoniques. Bien que généralement plus volumineuses que des sources à cathode froide, il peut être possible de les mettre en œuvre en acceptant un écart entre les sources et un pas d'hélice plus importants.

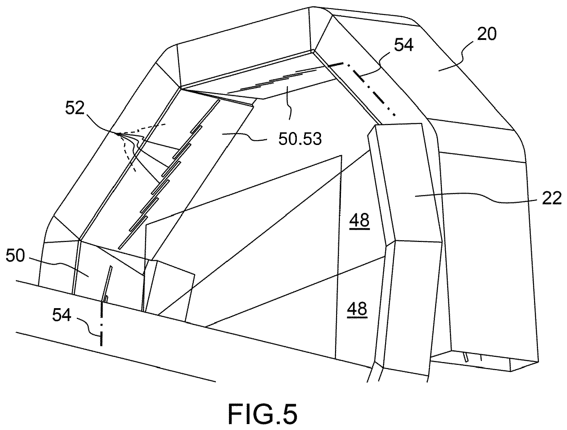

La figure 5 représente le générateur 20 et le détecteur 22 auxquels est associé un collimateur 50 possédant, en regard de chaque source, une fente 52 configurée pour obtenir le faisceau 48 en forme d'éventail. Le collimateur comprend un ou plusieurs plans 53 dans lequel les fentes 52 sont réalisées. Chaque plan 53 est parallèle à l'axe de translation 16. Les plans 53 appartiennent à une surface cylindrique d'axe 16. Dans la variante de la figure 5, chaque fente 52 n'est associée qu'à une seule source 24. Le collimateur 50 suit l'axe des sources 26 pour que chacune des fentes 52 soit en regard d'une seule source 24. Autrement dit, le collimateur 50 s'étend principalement selon un axe du collimateur 54 parallèle à l'axe des sources 26. Pour ne pas surcharger la figure 5, l'axe des sources 26, caché par le collimateur 50, n'est pas représenté. Les axes 26 et 54 sont parallèles entre eux.

Sur la figure 5, les fentes 52 associées aux différentes sources 24 s'étendent principalement selon des portions de droites parallèles entre elles. La sinusoïde évoquée plus haut est approximée à une droite.

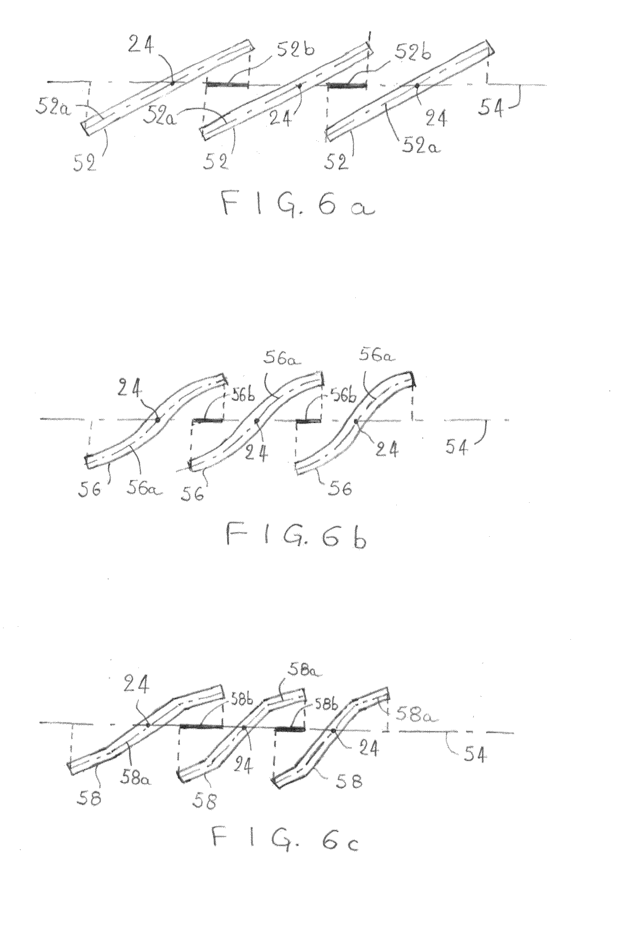

Les figures 6a, 6b et 6c représentent plusieurs exemples de formes de fentes du collimateur 50 réalisées dans un des plans 53 contenant l'axe du collimateur 54 et est perpendiculaire à un plan contenant l'axe des sources 26 et l'axe du collimateur 54. L'exemple de la figure 6a reprend les fentes 52 visibles sur la figure 5. Les fentes 52 s'étendent chacune selon une portion de droite. L'exemple de la figure 6b représente des fentes 56 s'étendant chacune selon une portion de sinusoïde et l'exemple de la figure 6c représente des fentes s'étendant chacune de plusieurs portions de droite formant une ligne brisée. Comme indiqué plus haut, la forme en portion de sinusoïde est la forme idéale des fentes permettant de réaliser le faisceau en éventail 48 adapté à une structure en hélice imbriquée du générateur et du détecteur. Il est cependant possible d'approximer la forme sinusoïdale à toute forme permettant aux faisceaux en éventail 48 de balayer le détecteur. Les formes représentées sur les figures 6a et 6c sont en portion de droite et sont plus faciles à réaliser qu'une forme sinusoïdale. L'invention peut être mise en œuvre quel que soit le nombre de portion de droites mis en œuvre pour approximer une courbe sinusoïdale.

De façon générale, Les différentes fentes 52 ou 56 ou 58 sont parallèles entre elles. Plus précisément, dans un même collimateur, les courbes selon lesquelles les fentes s'étendent principalement sont parallèles entre elles. Ceci permet aux différents faisceaux en éventail de rester parallèles entre eux.

Pour assurer une amplitude angulaire suffisante de la forme en éventail de chaque faisceau 48, les projections de fentes 52 consécutives sur l'axe 54 sont sécantes. Plus précisément, sur la figure 6a, chaque fente 52 s'étend principalement une portion de droite 52a. La portion de droite permet de réaliser le faisceau en éventail 48. La longueur de la portion de droite définit l'ouverture angulaire du faisceau 48 considéré. La largeur de la fente 52, définie perpendiculairement à la portion de droite 52a, donne l'épaisseur du faisceau 48 et est très faible par rapport à la longueur de la portion de droite.

Il est possible de projeter géométriquement, dans le plan 53, la portion de droite 52a sur l'axe 54, la projection se faisant perpendiculairement à l'axe 54. Les projections sur l'axe 54 de deux segments de droites 52a de deux fentes consécutives 52 possèdent une partie commune, repérée 52b en trait fort sur l'axe 54. Il en est de même sur la figure 6b où chaque fente 56 s'étend principalement selon une portion de sinusoïde 56a. Les projections sur l'axe 54 de portions de sinusoïde 56a de deux fentes consécutives 56 possèdent une partie commune 56b. Sur la figure 6c, chaque fente 58 s'étend principalement selon une ligne brisée 58a. Les projections sur l'axe 54 de lignes brisées 58a de deux fentes consécutives 58 possèdent une partie commune 58b. de façon générale, les fentes s'étendent principalement suivant des portions de courbes définies dans le plan 53 et les projections de portions de courbes correspondant à des fentes consécutives possèdent une partie commune.

En fonction de la géométrie du générateur 20 et du détecteur 22 et en fonction de l'amplitude angulaire souhaitée pour chaque faisceau 48, il est possible que les projections respectives sur l'axe 54 de plus de deux portions de courbes correspondant à plus de deux fentes consécutives possèdent des parties communes. Cependant, la forme en portion de droite ou en portion de sinusoïde suivie par les fentes 52 assure que les fentes elles-mêmes ne sont pas sécantes. Une telle forme des fentes serait impossible dans une réalisation où les sources seraient situées dans un premier plan perpendiculaire à l'axe 16 et où le détecteur serait situé dans un second plan parallèle au plan des sources. Avec deux plans parallèles, l'un pour les sources et l'autre pour le détecteur, les fentes auraient une forme d'arc de cercle et seraient donc sécantes. Les formes en hélices imbriquées des axes 26 et 28 permettent la réalisation de fentes voisines dont les projections sur l'axe des sources 26 se chevauchent mais dont les fentes elles-mêmes ne sont pas sécantes.

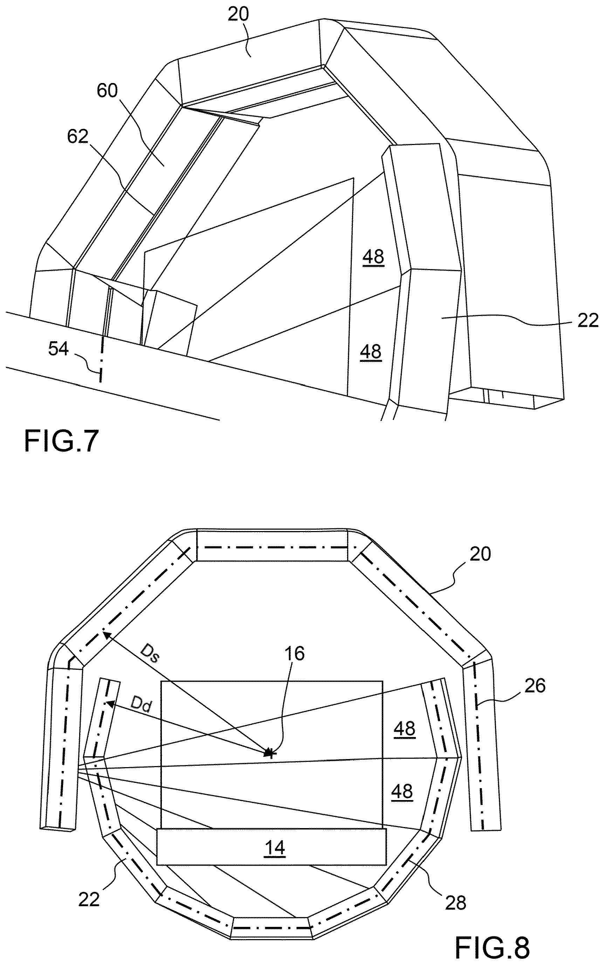

La figure 7 représente une variante de collimateur 60 comprenant une fente 62 associée à plusieurs sources 24. En effet, dans la variante de la figure 5, les fentes sont proches les unes des autres. En réduisant le pas des hélices, il est possible d'approximer les différentes fentes à une seule s'étendant selon l'axe 54. Dans la variante de la figure 7, l'axe des sources 26 et donc l'axe du collimateur 54 s'appuient sur un cylindre d'axe 16 à section de polygone régulier. Plusieurs sources 24 sont disposées sur un même côté du polygone. Le collimateur 60 comprend une fente 62 par côté du polygone et commune aux plusieurs sources 24 disposées le long de ce même côté.

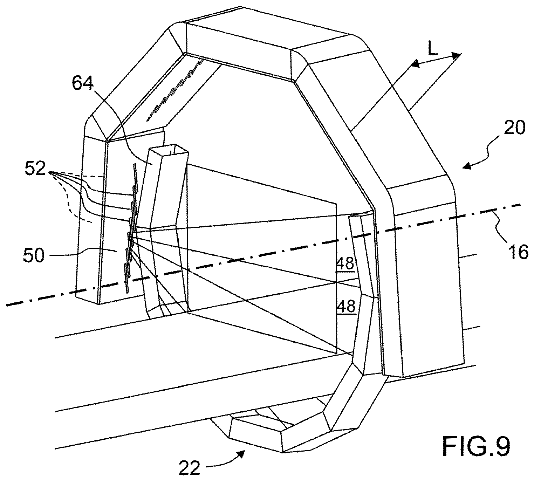

Les figures 8 et 9 représentent l'association d'un générateur 20 et d'un détecteur 22 dans lequel le détecteur 22 est disposé en partie entre le générateur 20 et l'axe 16 dans un plan perpendiculaire à l'axe 16 tout en laissant le passage libre pour le faisceau 48 issu du générateur 20 dans la zone où le détecteur 22 masque le générateur 20. Pour permettre cette disposition, la distance moyenne Ds de l'axe des sources 26 à l'axe 16 est plus grande que la distance moyenne Dd de l'axe du détecteur 28 à l'axe 16. Par distance moyenne on entend diamètre lorsque les hélices des axes des sources et du détecteur 26 et 28 s'appuient sur des cylindres à section circulaire. Lorsque les sections des cylindres sont des polygones réguliers, on peut définir la distance moyenne comme la moyenne de la distance d'un sommet et de la distance d'une côté du polygone. Il est possible de réaliser cette disposition relative du générateur et du détecteur pour d'autres formes plus complexes sur lesquelles les hélices peuvent s'appuyer.

Cette disposition du générateur et du détecteur permet de réduire le pas des hélices et donc de réduire les écarts des différents faisceaux par rapport à des plans perpendiculaires à l'axe 16, ce qui permet de faciliter la reconstruction de l'image. En effet, le générateur peut avoir une largeur importante, largeur définie parallèlement à l'axe 16 et représentée avec le repère L sur la figure 8. Cette largeur est notamment due à la présence de l'enceinte à vide 40 dont un exemple est donné sur la figure 4. L'axe des sources 26 est sensiblement situé à mi largeur du générateur 20. Dans la variante de la figure 1 où les distances moyennes Ds et Dd sont sensiblement égales, une des faces latérales du détecteur 22 est obligatoirement distant de l'axe des sources 28 d'au moins la moitié de la largeur L. Dans la variante des figures 8 et 9, il est possible de rapprocher la face latérale 64 du détecteur 22 de l'axe des sources 28 tout en permettant au faisceau 48 la possibilité de s'étendre sans toucher le détecteur 22 et notamment sa la face latérale 64. Sur la figure 8, un collimateur 50 est représenté.

Sur les figures 8 et 9, le détecteur 22 est représenté plus près de l'axe 16 que le générateur 20. L'inverse est également possible afin de réduire le pas des hélices. Cependant, la disposition des figures 8 et 9 présente l'avantage d'améliorer la qualité des images formées par le dispositif. En effet, dans cette disposition, le détecteur 22 est plus près du volume utile ce qui implique une plus faible dispersion du faisceau après avoir traversé le volume utile 18.

## Revendications

1. Dispositif de radiologie comprenant un support (14) mobile en translation selon un axe de translation (16) par rapport à un bâti (12) du dispositif (10), le support (14) étant destiné à porter un objet à imager, un générateur de rayons ionisants (20 ; 30) et un détecteur (22 ; 32) configuré pour détecter les rayons émis par le générateur (20 ; 30), le générateur (20 ; 30) et le détecteur (22 ; 32) étant en regard l'un par rapport à l'autre, le générateur (20 ; 30) comprenant plusieurs sources (24) dont on considère qu'elles émettent chacune à partir d'un point de focalisation, les points de focalisation des différentes sources (24) étant répartis le long d'un axe des sources (26 ; 36), le détecteur (22 ; 32) s'étendant selon un axe du détecteur (28 ; 38), axe des sources (26 ; 36) et l'axe du détecteur (28 ; 38) s'étendant en forme d'hélice imbriquées l'une dans l'autre autour de l'axe de translation (16), chacune des sources (24) émettant un faisceau de rayons ionisants (48) sensiblement plat et en forme d'éventail, le dispositif comprenant en outre un collimateur (50) possédant, en regard de chaque source (24), une fente (52) configurée pour obtenir le faisceau (48) en forme d'éventail réalisée dans un plan (53) du collimateur (50) parallèle à l'axe de translation (16), le collimateur (50) s'étendant principalement selon un axe du collimateur (54) parallèle à l'axe des sources (26 ; 36), les fentes (52) n'étant pas sécantes et **caractérisé en ce que** les fentes (52 ; 56 ; 58) s'étendent principalement chacune selon une portion de courbe (52a ; 56a ; 58a) définie dans le plan (53) du collimateur (50) et **en ce que** des projections, perpendiculairement à l'axe du collimateur (54), de portions de courbes, correspondant à des fentes consécutives, sur l'axe du collimateur (54) possèdent une partie commune (52b ; 56b ; 58b).

2. Dispositif de radiologie selon la revendication 1, **caractérisé en ce que** le générateur (20 ; 30) et le détecteur (22 ; 32) sont fixes par rapport au bâti (12).

3. Dispositif de radiologie selon l'une des revendications précédentes, **caractérisé en ce que** les formes en hélice sont positionnées entre elles de façon à ce que pour chacune des sources (24) un faisceau (48) de rayons ionisants issu de la source considérée et atteignant une portion du détecteur (22 ; 32) soit sensiblement situé dans un plan perpendiculaire à l'axe de translation (16).

4. Dispositif de radiologie selon la revendication 3, **caractérisé en ce que** les portions de courbes (52a ; 56a ; 58a) suivent sensiblement une forme sinusoïdale.

5. Dispositif de radiologie selon la revendication 4, **caractérisé en ce que** chaque portion de courbe comprend au moins une portion de droite (52a ; 58a) approximant la forme sinusoïdale.

6. Dispositif de radiologie selon l'une des revendications précédentes, **caractérisé en ce que** les portions de courbe (52a ; 56a ; 58a) des différentes fentes (52 ; 56 ; 58) s'étendent parallèlement entre elles.

7. Dispositif de radiologie selon l'une des revendications précédentes, **caractérisé en ce que** le détecteur (22 ; 32) est disposé en partie entre le générateur (20 ; 30) et l'axe de translation (16) dans un plan perpendiculaire à l'axe de translation (16) tout en laissant un passage libre pour les rayons émis par le générateur (20 ; 30) en direction du détecteur (22 ; 32).

8. Dispositif de radiologie selon l'une des revendications précédentes, **caractérisé en ce que** la forme en hélice de l'axe des sources (26 ; 36) et la forme en hélice de l'axe du détecteur (28, 38) s'appuient chacune sur une courbe régulière centrée sur l'axe de translation (16).

9. Dispositif de radiologie selon la revendication 5, **caractérisé en ce que** les courbes régulières sont formées chacune sur une surface cylindrique à section circulaire dont l'axe est l'axe de translation (16).

10. Dispositif de radiologie selon la revendication 5, **caractérisé en ce que** les courbes régulières sont formées chacune sur une surface cylindrique à section polygonale régulière dont l'axe est l'axe de translation (16).

11. Dispositif selon l'une des revendications précédentes, **caractérisé en ce que** chaque source (24) comprend une cathode froide (38) émettant un faisceau d'électrons (46) par effet de champ.

## Patentansprüche

1. Radiologievorrichtung, umfassend einen Träger (14), der entlang einer Translationsachse (16) in Bezug auf ein Gestell (12) der Vorrichtung (10) translatorisch beweglich ist, wobei der Träger (14) dazu bestimmt ist, ein abzubildendes Objekt zu tragen, einen Generator ionisierender Strahlen (20; 30) und einen Detektor (22; 32) , der so konfiguriert ist, dass er die vom Generator (20; 30) emittierten Strahlen erfasst, wobei der Generator (20; 30) und der Detektor (22; 32) einander gegenüberliegen, wobei der Generator (20; 30) mehrere Quellen (24) umfasst, von denen angenommen wird, dass sie jeweils von einem Brennpunkt aus emittieren, wobei die Brennpunkte der verschiedenen Quellen (24) entlang einer Achse der Quellen (26; 36) verteilt sind, wobei sich der Detektor (22; 32) entlang einer Achse des Detektors (28; 38) erstreckt, wobei sich die Achse der Quellen (26; 36) und die Achse des Detektors (28; 38) in Form ineinander verschachtelter Schrauben um die Translationsachse (16) erstrecken, wobei jede der Quellen (24) einen im Wesentlichen flachen und fächerförmigen Strahl ionisierender Strahlen (48) emittiert, wobei die Vorrichtung ferner einen Kollimator (50) umfasst, der gegenüber jeder Quelle (24) einen Schlitz (52) aufweist, der so konfiguriert ist, dass er den fächerförmigen Strahl (48) erhält, und der in einer zur Translationsachse (16) parallelen Ebene (53) des Kollimators (50) erstellt ist, wobei sich der Kollimator (50) hauptsächlich entlang einer Achse des Kollimators (54) parallel zur Achse der Quellen (26; 36) erstreckt, wobei die Schlitze (52) sich nicht schneiden, und **dadurch gekennzeichnet, dass** die Schlitze (52; 56; 58) sich jeweils hauptsächlich entlang eines Kurvenabschnitts (52a; 56a; 58a) erstrecken, der in der Ebene (53) des Kollimators (50) definiert ist, und dass Vorsprünge, senkrecht zur Achse des Kollimators (54), von Kurvenabschnitten, die aufeinanderfolgenden Schlitzen entsprechen, auf die Achse des Kollimators (54) einen gemeinsamen Teil (52b; 56b; 58b) aufweisen.

2. Radiologievorrichtung nach Anspruch 1, **dadurch gekennzeichnet, dass** der Generator (20; 30) und der Detektor (22; 32) in Bezug auf das Gestell (12) feststehend sind.

3. Radiologievorrichtung nach einem der vorhergehenden Ansprüche, **dadurch gekennzeichnet, dass** die Schraubenformen so zueinander positioniert sind, dass für jede der Quellen (24) ein Strahl (48) ionisierender Strahlen, der von der betrachteten Quelle ausgeht und einen Abschnitt des Detektors (22; 32) erreicht, im Wesentlichen in einer Ebene senkrecht zur Translationsachse (16) liegt.

4. Radiologievorrichtung nach Anspruch 3, **dadurch gekennzeichnet, dass** die Kurvenabschnitte (52a; 56a; 58a) im Wesentlichen einer Sinusform folgen.

5. Radiologievorrichtung nach Anspruch 4, **dadurch gekennzeichnet, dass** jeder Kurvenabschnitt mindestens einen geraden Abschnitt (52a; 58a) umfasst, der sich der Sinusform annähert.

6. Radiologievorrichtung nach einem der vorhergehenden Ansprüche, **dadurch gekennzeichnet, dass** sich die Kurvenabschnitte (52a; 56a; 58a) der verschiedenen Schlitze (52; 56; 58) parallel zueinander erstrecken.

7. Radiologievorrichtung nach einem der vorhergehenden Ansprüche, **dadurch gekennzeichnet, dass** der Detektor (22; 32) teilweise zwischen dem Generator (20; 30) und der Translationsachse (16) in einer Ebene senkrecht zur Translationsachse (16) angeordnet ist, während er einen freien Durchgang für die vom Generator (20; 30) in Richtung des Detektors (22; 32) emittierten Strahlen lässt.

8. Radiologievorrichtung nach einem der vorhergehenden Ansprüche, **dadurch gekennzeichnet, dass** die Schraubenform der Achse der Quellen (26; 36) und die Schraubenform der Achse des Detektors (28, 38) jeweils auf einer regelmäßigen Kurve zentriert auf der Translationsachse (16) aufliegen.

9. Radiologievorrichtung nach Anspruch 5, **dadurch gekennzeichnet, dass** die regelmäßigen Kurven jeweils auf einer Zylinderfläche mit kreisförmigem Querschnitt gebildet sind, deren Achse die Translationsachse (16) ist.

10. Radiologievorrichtung nach Anspruch 5, **dadurch gekennzeichnet, dass** die regelmäßigen Kurven jeweils auf einer zylindrischen Fläche mit regelmäßigem polygonalem Querschnitt gebildet sind, deren Achse die Translationsachse (16) ist.

11. Vorrichtung nach einem der vorhergehenden Ansprüche, **dadurch gekennzeichnet, dass** jede Quelle (24) eine kalte Kathode (38) umfasst, die durch Feldeffekt einen Elektronenstrahl (46) emittiert.

## Claims

1. A radiology device comprising a support (14) capable of translational movement along an axis of translation (16) relative to a frame (12) of the device (10), the support (14) being intended to support an object that is to be imaged, an ionising-ray generator (20; 30) and a detector (22; 32) configured to detect the rays emitted by the generator (20; 30), the generator (20; 30) and the detector (22; 32) facing one another, the generator (20; 30) comprising several sources (24) each considered to emit from a focal point, the focal points of the various sources (24) being distributed along an axis of the sources (26; 36), the detector (22; 32) extending along an axis of the detector (28; 38), the axis of the sources (26; 36) and the axis of the detector (28; 38) extending in the form of mutually intertwined helices about the axis of translation (16), each of the sources (24) emitting a substantially flat and fan-shaped beam of ionising rays (48), the device further comprising a collimator (50) having, facing each source (24), a slot (52) configured to obtain the fan-shaped beam (48) and produced in a plane (53) of the collimator (50) that is parallel to the axis of translation (16), the collimator (50) extending mainly along an axis of the collimator (54) parallel to the axis of the sources (26; 36), wherein the slots (52) are not secant and **characterised in that** the slots (52; 56; 58) each extend mainly along a curve portion (52a; 56a; 58a) defined in the plane (53) of the collimator (50) and **in that** the projections, perpendicular to the axis of the collimator (54), of curve portions, corresponding to consecutive slots, onto the axis of the collimator (54), have a part (52b; 56b; 58b) in common.

2. The radiology device according to claim 1, **characterised in that** the generator (20; 30) and the detector (22; 32) are fixed relative to the frame (12).

3. The radiology device according to one of the preceding claims, **characterised in that** the helical shapes are positioned relative to one another in such a way that, for each of the sources (24), a beam (48) of ionising rays emanating from the source concerned and reaching a portion of the detector (22; 32) is situated substantially in a plane perpendicular to the axis of translation (16).

4. The radiology device according to claim 3, **characterised in that** the curve portions (52a; 56a; 58a) substantially follow a sinusoidal shape.

5. The radiology device according to claim 4, **characterised in that** each curve portion comprises at least one straight portion (52a; 58a) approximating the sinusoidal shape.

6. The radiology device according to one of the preceding claims, **characterised in that** the curve portions (52a; 56a; 58a) of the various slots (52; 56; 58) extend parallel to one another.

7. The radiology device according to one of the preceding claims, **characterised in that** the detector (22; 32) is arranged partly between the generator (20; 30) and the axis of translation (16) in a plane perpendicular to the axis of translation (16) while leaving a free space for the passage of the rays emitted by the generator (20; 30) toward the detector (22; 32).

8. The radiology device according to one of the preceding claims, **characterised in that** the helical shape of the axis of the sources (26; 36) and the helical shape of the axis of the detector (28, 38) are each based on a regular curve centred on the axis of translation (16).

9. The radiology device according to claim 5, **characterised in that** the regular curves are each formed on a cylindrical surface with the cross section of a circle, the axis of which is the axis of translation (16).

10. The radiology device according to claim 5, **characterised in that** the regular curves are each formed on a cylindrical surface with the cross section of a regular polygon, the axis of which is the axis of translation (16).

11. The device according to one of the preceding claims, **characterised in that** each source (24) comprises a cold cathode (38) emitting an electron beam (46) through a field effect.
